# EUROPEAN PATENT APPLICATION

(11) **EP 1 917 979 A1**
(43) Date of publication of application: **07.05.2008**
(21) Application number: 06796612.7
(22) Date of filing: 22.08.2006
(51) Int. Cl.: A61K 45/00, A61K 31/4725, A61P 9/06, C07D 401/06

(54) **THERAPEUTIC AGENT FOR ATRIAL FIBRILLATION**

(30) Priority: 23.08.2005 JP 2005241403
(71) Applicant: Astellas Pharma Inc., Tokyo 103-8411 (JP)
(72) Inventor: WADA, Koichi, Tokyo; 1038411 (JP); MASUDA, Noriyuki, Tokyo; 1038411 (JP); TANIGUCHI, Keiichi, Tokyo; 1038411 (JP)
(74) Representative: Bates, Philip Ian
(86) International application number: PCT/JP2006/316349
(87) International publication number: WO 2007/023775

(57) **Abstract**

Disclosed is a therapeutic agent for atrial fibrillation comprising an I_{f} current inhibitor, particularly (-)-N-[2-[(R)-3-(6,7-dimethoxy-1,2,3,4-tetrahydro- isoquinoline-2-carbonyl)piperidino]ethyl]-4-fluorobenzamide monophosphate, as an active ingredient. This active ingredient has more preferred properties for use as a therapeutic agent for atrial fibrillation compared to verapamil (a Ca antagonist) and atenolol (a β-blocker) which have been conventionally used as the therapeutic agents for atrial fibrillation.

## Description

### Technical Field

This invention relates to a drug, in particular, an agent for treating atrial fibrillation, which comprises an If current inhibitor as an active ingredient. More particularly, the present invention relates to an agent for treating atrial fibrillation, which comprises (-)-N-{2-[(R)-3-(6,7-dimethoxy-1,2,3,4-tetrahydroisoquinoline-2-carbonyl)piperidino]ethyl}-4-fluorobenzamide monophosphate as an active ingredient.

### Background Art

In Japan, the prevalence of atrial fibrillation increases with advancing age and it is estimated about 6% of people aged 65 or over suffer from atrial fibrillation. Atrial fibrillation means a clinical state with irregular and highly frequent atrial excitation. Although atrial fibrillation differs from lethal arrhythmia, it should be strictly controlled since it is accompanied by the onset of cardiac disturbance due to persistent tachycardia and the appearance of subjective symptoms and, in its turn, seriously worsens the quality of life (QOL). Furthermore, blood congestion evokes atrial thrombosis which causes life-threatening cerebral embolism. If surviving, there are nervous disorders such as paralysis as aftereffects, which further worsens the QOL. This is a serious problem to be overcome in Japan's aging society.
Atrial fibrillation is generally classified into paroxysmal atrial fibrillation, persistent atrial fibrillation and permanent atrial fibrillation depending on the duration of fibrillation. In patients with permanent atrial fibrillation, it is particularly difficult to restore the sinus rhythm. The irregular atrial excitation is conducted to the ventricles so that the ventricles irregularly and highly frequently contract persistently. Therefore, a therapy for partly blocking the pulse conduction in the atrioventricular node existing between the atrium and the ventricle with the use of an agent for treating atrial fibrillation has been applied and thus making the ventricular rate regular and less frequent (ventricular rate control). In these days, β-blockers which suppress the atrioventricular node function (typified by Atenolol) or Ca antagonists (typified by Verapamil) have been employed in the ventricular rate control. However, these existing drugs have a problem of affecting the systemic hemodynamics, for example, having a negative inotropism or a hypotension. The term "sinus rhythm" as used herein means the state where the heart beats under the pace making action of sinoatrial node.
It has been found that there is an ion current called If current, which is composed of the permeation of sodium ion and potassium ion and is activated by the hyperpolarization of the membrane potential or stimulation with β-receptor, in pacemaker cells of the cardiac sinoatrial node (Difrancesco D. et al., J. Physiol. 377:61-88, 1986; Irisawa H., et al., Physiol. Rev. 73:197-227, 1993; and DiFracesco D., Annu. Rev. Physiol., 55:455-472, 1993). It is known that this If current causes spontaneous electrical excitation of the sinoatrial node. Although it has been known that an If current inhibitor is useful as a heart rate-lowering agent because of lowering the frequency of the electrical excitation, it has never been known that such an If current inhibitor is useful as an agent for treating atrial fibrillation, which suppresses the atrioventricular node function and controls the ventricular rate in atrial fibrillation. Patent Document 1 discloses that an isoquinoline derivative or its salt serving as an If current inhibitor has an effect of lowering the heart rate. However, this document neither discloses that these compounds are useful in treating atrial fibrillation nor presents any pharmacological data suggesting that these compounds would be usable in treating atrial fibrillation.

Patent Document 1: WO 00/75133

### Disclosure of the Invention

### Problems that the Invention is to Solve

An object of the present invention is to provide a drug comprising an If current inhibitor as an active ingredient which is expected as being useful as an agent for treating atrial fibrillation while showing no side effect. In particular, the present invention aims at providing an agent for treating atrial fibrillation, which comprises (-)-N-{2-[(R)-3-(6,7-dimethoxy-1,2,3,4-tetrahydroisoquinoline-2-carbonyl)piperidino]ethyl}-4-fluorobenzamide monophosphate as an active ingredient.

### Means for Solving the Problems

As a result of extensive studies on If current inhibitors, the present inventors have confirmed that the If current inhibitors are useful as agents for treating atrial fibrillation, thereby completing the present invention. They have found out that (-)-N-{2-[(R)-3-(6,7-dimethoxy-1,2,3,4-tetrahydroisoquinoline-2-carbonyl)piperidino]ethyl}-4-fluorobenzamide monophosphate (hereinafter sometimes referred to as "compound A") represented by the following structural formula is particularly useful as an agent for treating atrial fibrillation, thereby completing the present invention. Thus, the present invention relates to an agent for treating atrial fibrillation, comprising an If current inhibitor as an active ingredient, in particular, an agent for treating atrial fibrillation, comprising compound A as an active ingredient.

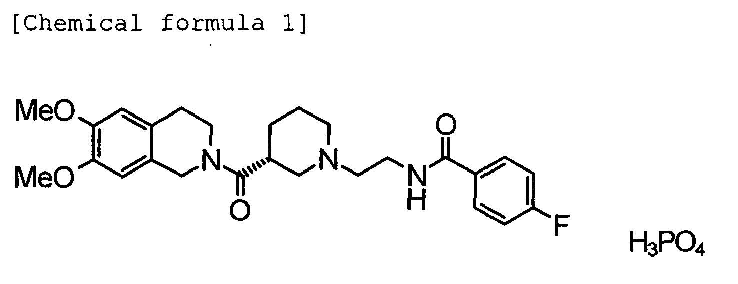

### Advantage of the Invention

The present invention is useful as providing an agent for treating atrial fibrillation.

### Best Mode for Carrying Out the Invention

In a preferred embodiment, the present invention relates to an agent for treating atrial fibrillation, comprising an If current inhibitor as an active ingredient. In a more preferred embodiment, it relates to an agent for treating atrial fibrillation, comprising compound A as an active ingredient. More specifically, the present invention is as follows.
[1] An agent for treating atrial fibrillation, which comprises an If current inhibitor as an active ingredient.
[2] The agent for treating atrial fibrillation according to the above [1], wherein the If current inhibitor is a compound selected from (-)-N-{2-[(R)-3-(6,7-dimethoxy-1,2,3,4-tetrahydroisoquinoline-2-carbonyl)piperidino]ethyl}-4-fluorobenzamide, Zatebradine, Ivabradine, and salts thereof.
[3] An agent for treating atrial fibrillation, which comprises (-)-N-{2-[(R)-3-(6,7-dimethoxy-1,2,3,4-tetrahydroisoquinoline-2-carbonyl)piperidino]ethyl}-4-fluorobenzamide or a salt thereof as an active ingredient.
[4] An agent for treating atrial fibrillation, which comprises (-)-N-{2-[(R)-3-(6,7-dimethoxy-1,2,3,4-tetrahydroisoquinoline-2-carbonyl)piperidino]ethyl}-4-fluorobenzamide monophosphate as an active ingredient.
Next, the present invention will be described in greater detail.
The term "I_{f} current inhibitor" means a compound inhibiting an If current. As examples thereof, If current inhibitors such as compound A, Zatebradine and Ivabradine can be cited. Compound A is most preferable as the If current inhibitor.
The term "agent for treating atrial fibrillation" means a drug which suppresses the atrioventricular node and
maintains the ventricular rate in a normal state while atrial fibrillation persists.

Compound A can be easily obtained by using the method described in Patent Document 1 or other production methods in accordance therewith. Zatebradine can be easily obtained by using the method described in U.S. Patent 4490369, while Ivabradine can be easily obtained by using the method described in European Patent 534859 or other production methods in accordance therewith.
Although compound A is monophosphate, (-)-N-{2-[(R)-3-(6,7-dimethoxy-1,2,3,4-tetrahydroisoquinoline-2-carbonyl)piperidino]ethyl}-4-fluorobenzamide can form salts together with other acids in addition to phosphate. Such salts are included in the scope of the present invention as long as being pharmaceutically acceptable. Specific examples thereof include a salt with an inorganic acid such as hydrobromic acid, hydroiodic acid, hydrochloric acid, sulfuric acid or nitric acid and a salt with an organic acid such as formic acid, acetic acid, propionic acid, oxalic acid, malonic acid, succinic acid, fumaric acid, maleic acid, malic acid, tartaric acid, citric acid, methanesulfonic acid, ethanesulfonic acid, p-toulenesulfonic acid, aspartic acid or glutamic acid. (-)-N-{2-[(R)-3-(6,7-Dimethoxy-1,2,3,4-tetrahydroisoquinoline-2-carbonyl)piperidino]ethyl}-4-fluorobenzamide include various hydrates, solvates and crystalline polymorphism of the free compound of a pharmaceutically acceptable salt thereof.

The preparation according to the present invention can be formulated by a conventionally employed method with the use of a drug carrier, an excipient and so on commonly employed in the art. Administraiton may be made either by oral administration in the form of a tablet, a pill, a capsule, granules, powder, a liquid preparation or the like or by parenteral administration in the form of an injection such as an intraarticular, intravenous or intramuscular injection, a suppository, an eye drop, an ophthalmic ointment, a transdermal liquid preparation, an ointment, a transdermal patch, a transmucosal liquid preparation, a transmucosal patch, an inhalation or the like.
As a solid composition for oral administration according to the present invention, a tablet, a dust, granules or the like may be used. In such a solid composition, one or more active ingredients are mixed with at least one inert diluent, for example, lactose, mannitol, glucose, hydroxypropylcellulose, microcrystalline cellulose, starch, polyvinylpyrrolidone and/or magnesium metasilicate aluminate. In accordance with the conventional method, the composition may further contain an additive other than the inert diluent, for example, a lubricant such as magnesium stearate, a disintegrating agent such as calcium cellulose glycolate, a stabilizer or a dissolution aid. If necessary, a tablet or a pill may be coated with a sugar coating made of, for example, sucrose, gelatin, hydroxypropylcellulose or hydroxypropylmethylcellulose phthalate or a gastric- or enteric-coating film.
A liquid composition for oral administration includes an emulsion, a solution, a suspension, a syrup, an elixir and so on each being pharmaceutically acceptable. The liquid composition contains an inert diluent commonly employed, for example, purified water or ethanol. In addition to the inert diluent, the liquid composition may contain an auxiliary such as a solubilizer, a humectant or a suspending agent, a sweetener, a flavor, a fragrance or an antiseptic.

An injection for parenteral administration contains an aseptic water based or non-water based solvent, a suspending agent or an emulsifying agent. Examples of the water based solvent include distilled water for injection and physiological saline. Examples of the non-water based solvent or the suspending agent include propylene glycol, polyethylene glycol, a vegetable oil such as olive oil, an alcohol such as ethanol, Polysorbate 80 [name in Pharmacopeia] and so on. Such a composition may further contain a tonicity agent, an antiseptic, a humectant, an emulsifier, a dispersant, a stabilizer or a dissolution aid. The composition is sterilized by, for example, filtering through a bacteria-retaining filter, adding a bactericide or irradiating. It is also possible that an aseptic solid composition is prepared and then dissolved or suspended in an aseptic vehicle for injection before using.
As a transmucosal preparation such as a nasal preparation, use is made of a solid, liquid or semisolid preparation that can be produced in accordance with a publicly known method. For example, it is prepared by appropriately adding a publicly known pH adjusting agent, an antiseptic, a thickener or an excipient to give a mixture in a solid, liquid or semisolid state. The nasal preparation is administered by using a spray, a nasal dripping device, a tube, an intranasal delivering device or the like.
The drug to be used in the present invention is administered to a patient suffering from atrial fibrillation. In oral administration, the daily dose is usually from about 0.01 to 1000 mg, preferably from 0.1 to 300 mg/kg, more preferably from 0.1 to 100 mg and the administration is conducted one or two to four times per day. In intravenous administration, it is suitable that the daily dose is from about 0.001 to 100 mg per kg body weight and the administration is conducted one or multiple times per day. The dose is appropriately determined in each case by taking the conditions, age, sex and so on of the patient into consideration.

### [Examples]

Next, the present invention will be described in greater detail by reference to the following Examples.

### Example 1

### Test of the suppression of canine cardiac atrioventricular node function

### 1. Test method

Male beagle dogs were employed in this test. Under halothane anesthesia, an electrode catheter was inserted from the left femoral artery and the front end thereof was placed in the non-coronary cusp of His bundle to thereby record the His bundle electrocardiogram. A pacing catheter was inserted via a sheath introducer placed in the right femoral vein and the front end thereof was fixed to the inner wall of the right atrium. Electrodes for recording body surface electrocardiogram were attached to the four limbs and thus the second-lead electrocardiogram was recorded. A drug was administered via the sheath introducer placed in the left femoral vein.

### 2. Drugs

Compound A and Ivabradine were intravenously administered in a dose of 0.1 mg/kg. Verapamil, which was used as a comparative compound, was intravenously administered in a dose of 0.1 mg/kg. A group to which a vehicle was administered was referred to as the control group.

### 3. Evaluation items and statistical treatment

After the stabilization period following the surgical operation, AH intervals in the His bundle electrocardiograms were measured in the sinus rhythm and during pacing the right atrium at intervals of 400 msec and 300 msec. One hour after the intravenous administration of 0.1 mg/kg of compound A, the AH intervals in the His bundle electrocardiogram as described above were measured. In the case of the comparative drug Verapamil, the AH intervals in the His bundle electrocardiogram as described above were measured before the administration and 30 minutes after the administration. The effect of a compound was expressed in the change in the AH intervals in the individual His bundle electrocardiograms in the sinus rhythm and during pacing the right atrium at intervals of 400 msec and 300 msec from the value before the administration of the drug and the data were compared with the data of the control group (Student's t-test) at a significant level of 5%. The term "AH interval in His bundle electrocardiogram" means the period of time wherein an electrical excitation generated at the atrium passes the atrioventricular node and arrives at His bundle.

### 4. Results and Discussion

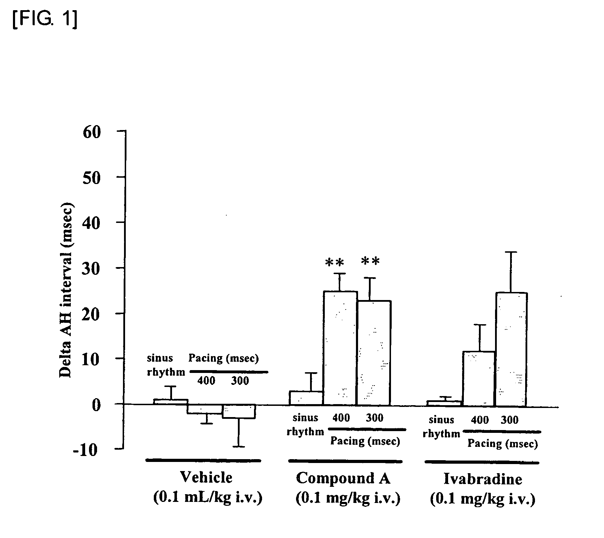

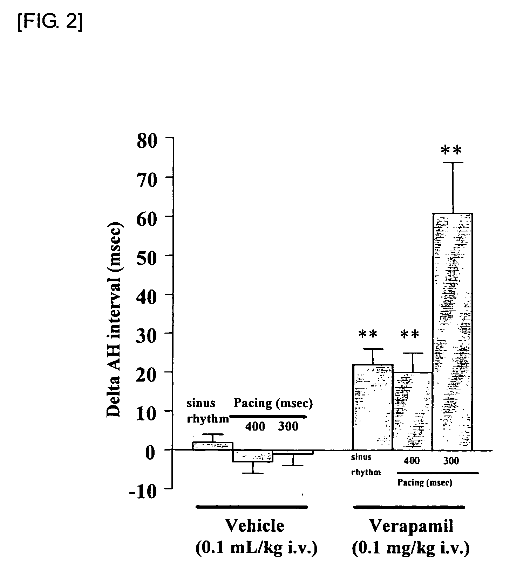

Fig. 1 shows the results. AH interval in the His bundle electrocardiogram is an indication that shows the pulse conductive function of the atrioventricular node. The administration of compound A exerted no effect on the AH interval in the His bundle electrocardiogram in the sinus rhythm but the AH intervals in the His bundle electrocardiograms during high-frequent stimulation by pacing were prolonged at a significant level of 1%. These results indicate that compound A suppressed the cardiac atrioventricular node function under the high-frequent stimulation conditions. Ivabradine totally showed similar results to compound A, though no significant difference at a significant level of 5% was observed. However, it is considered that compound A has a preferred profile to Ivabradine, since it significantly prolonged the AH intervals at a significant level of 1% during pacing the right atrium at intervals of 400 msec and 300 msec. Since atrial fibrillation means a pathological situation accompanied by atrial excitation at a high frequency, compound A can control the ventricular rate within an appropriate range through the suppression of the cardiac atrioventricular node function at atrial fibrillation.
On the other hand, Fig. 2 shows the results of a Ca antagonist Verapamil employed as a comparative compound. Verapamil significantly prolonged the AH interval in the His bundle electrocardiograms both in the sinus rhythm and during the high-frequent stimulation. It is known that Verapamil induces atrioventricular block in clinical practice, which is seemingly caused by the suppression of the cardiac atrioventricular node during the sinus rhythm too.
In summary, compound A and Ivabradine suppress the cardiac atrioventricular node function during the high-frequent stimulation but do not suppress the atrioventricular node function in the sinus rhythm. This pharmacological characteristic clearly differs from that of the existing drug Verapamil. Accordingly, it is considered that compound A and Ivabradine can control the ventricular rate in atrial fibrillation without causing atrioventricular block and, therefore, they are expected as useful as agents for treating atrial fibrillation that are useful in clinical practice.

### Example 2

### Test of measurement of anesthetized swine ventricular rate

### 1. Test method

Male pigs (Landrace/White F1) were employed in this test. Under pentbarbital anesthesia, a thoracotomy was performed at the fourth right intercostal space and an electrode for electrical stimulation was attached to the right atrial surface thus exposed. Electrodes for recording body surface electrocardiogram were attached to the four limbs and thus the second-lead electrocardiogram was recorded. A catheter for drug administration was placed in the right femoral vein.

### 2. Drugs

Compound A was intravenously administered in a dose of 0.3 mg/kg. Atenolol, which was a β-blocker used as a comparative compound, was intravenously administered in a dose of 1 mg/kg. A group to which a vehicle was administered was referred to as the control group.

### 3. Evaluation items and statistical treatment

A 50 Hz burst stimulation was applied to the right atrium via the electrode for electrical stimulation and the ventricular rate per minute was measured as R-waves in the body surface electrocardiogram. Thirty minutes after the administration of compound A or the comparative drug, a 50 Hz burst stimulation was applied again to the right atrium and then the R-waves per minute in the body surface electrocardiogram were measured. The effect of a compound was expressed in the change in the R waves per minute in the body surface electrocardiogram from the value before the administration of the drug and the data were compared with the data of the control group (Student's t-test) at a significant level of 5%.

### 4. Results and Discussion

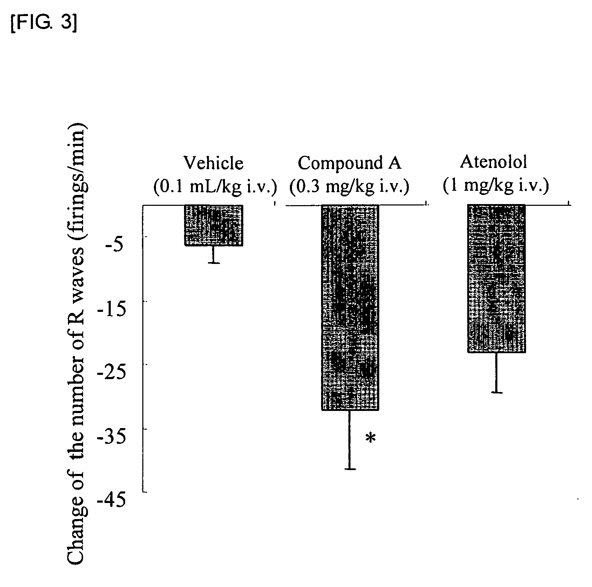

Fig. 3 shows the results. Compound A (0.3 mg/kg) significantly decreases the ventricular rate during the 50 Hz burst stimulation. This result was interpreted as follows. Namely, compound A suppressed the atrioventricular node and partly prevented the high-frequent atrial excitation from conduction to the ventricle, which caused a decrease in the ventricular rate. The ventricular rate-controlling effect of compound A was sufficiently exerted in a dose equivalent to 1/3 of Atenolol (1 mg/kg). Accordingly, it is considered that compound A is clinically efficacious in controlling the ventricular rate in atrial fibrillation and, therefore, is useful as an agent for treating atrial fibrillation. It is also considered that the efficacy of compound A is stronger by thrice or more than the existing drug Atenolol.

As discussed above, it is considered that compound A is clinically efficacious in treating atrial fibrillation since it is expected as having less side effect than Verapamil that is a Ca antagonist having been employed as an agent for treating atrial fibrillation and has more preferred properties compared with Atenolol which is a β-blocker.

### Brief Description of the Drawings

[Fig. 1] Fig. 1 shows the effect of compound A on the AH intervals in the His bundle electrocardiograms of anesthetized dogs. In Fig. 1, "sinus rhythm" stands for the sinus rhythm. "Pacing" stands for the atrial pacing (electrical stimulation). "400" and "300" in "Pacing (msec)" respectively stand for the states of pacing the atrium at intervals of 400 and 300 msec. "msec" stands for 1/1000 sec. "Delta AH interval (msec)" stands for a change in the AH intervals in the His bundle electrocardiograms before and after the administration of compound A or Ivabradine. "Vehicle" means a control group with the administration of the vehicle, "Compound A" means "compound A" and "Ivabradine" means Ivabradine. "**" means being significantly different from the control group at a risk less than 1%.
[Fig. 2] Fig. 2 shows the effect of Verapamil (a comparative drug) on the AH intervals in the His bundle electrocardiograms of anesthetized dogs. In Fig. 2, "Verapamil" stands for Verapamil. Other symbols respectively have the same meanings as in Fig. 1.
[Fig. 3] Fig. 3 shows the effect of compound A on the ventricular rate of anesthetized pigs under the atrial burst stimulation. In Fig. 3, "Change of the number of R waves (firings/min)" stands for a change in the number of R waves per minute in the body surface electrocardiograms before and after the drug administration. "Atenolol" stands for Atenolol. "**" means being significantly different from the control group at a risk less than 5%. Other symbols respectively have the same meanings as in Fig. 1.

## Claims

1. An agent for treating atrial fibrillation, which comprises an If current inhibitor as an active ingredient.

2. The agent for treating atrial fibrillation according to claim 1, wherein the If current inhibitor is a compound selected from (-)-N-{2-[(R)-3-(6,7-dimethoxy-1,2,3,4-tetrahydroisoquinoline-2-carbonyl)piperidino]ethyl}-4-fluorobenzamide, Zatebradine, Ivabradine, and salts thereof.

3. An agent for treating atrial fibrillation, which comprises (-)-N-{2-[(R)-3-(6,7-dimethoxy-1,2,3,4-tetrahydroisoquinoline-2-carbonyl)piperidino]ethyl}-4-fluorobenzamide or a salt thereof as an active ingredient.

4. An agent for treating atrial fibrillation, which comprises (-)-N-{2-[(R)-3-(6,7-dimethoxy-1,2,3,4-tetrahydroisoquinoline-2-carbonyl)piperidino]ethyl}-4-fluorobenzamide monophosphate as an active ingredient.
